# EUROPÄISCHE PATENTSCHRIFT

(11) **EP 0 539 348 B1**
(45) Veröffentlichungstag und Bekanntmachung des Hinweises auf die Patenterteilung: **06.09.1995**
(21) Anmeldenummer: 92890215.4
(22) Anmeldetag: 13.10.1992
(51) Int. Cl.: A61M 5/315

(54) **Einwegspritze**
Single-use syringe
Séringe à usage unique

(30) Priorität: 22.10.1991 AT 2102/91
(43) Veröffentlichungstag der Anmeldung: 28.04.1993
(73) Patentinhaber: Wimmer, Erwin, A-4300 St. Valentin (AT)
(72) Erfinder: Wimmer, Erwin, A-4300 St. Valentin (AT)
(74) Vertreter: Hübscher, Heiner, Dipl.-Ing.

(56) Entgegenhaltungen:
- WO-A-84/02278
- DE-A- 2 025 379
- DE-A- 4 017 920
- FR-A- 2 291 702
- US-A- 4 266 557

## Beschreibung

Die Erfindung bezieht sich auf eine Einwegspritze aus Spritzenzylinder und Spritzenkolben, welcher einerends offene Spritzenzylinder andernends eine bis auf einen Nadelansatz geschlossene Stirnwand aufweist und welcher Spritzenkolben einen an seinem freien, aus dem Spritzenzylinder herausragenden Ende eine Handhabe tragenden Kolbenschaft und innerhalb des Spritzenzylinders einen Kolbenkopf und eine über einen umlaufenden Kragenteil am Kolbenkopf sitzende, an der Zylinderinnenfläche anliegende Ringdichtung umfaßt, wobei der Kragenteil in Form eines sich in axialer Richtung zum Kolbenschaft hin erweiternden Hohlkegelstumpfes ausgebildet ist und der radiale Abstand der Mantelinnenfläche des hohlkegelstumpfförmigen Kragenteils in axialer Richtung zum Kolbenschaft hin stetig zunimmt, an welchen Kragenteil sich die in Form eines koaxialen Zylinders ausgebildete Ringdichtung, kolbenkopfseitig frei vorstehend, anschließt.

Solche aus der FR-A 22 91 702 bekannten Einwegspritzen sind durchwegs aus Kunststoff hergestellt und gewährleisten auf Grund der sich durch ihre spezielle Kolbenausbildung ergebenden weichen radialen Abstützung der Dichtung und des damit verbundenen federnden Dichtungssystems einerseits beim Saug- und Druckhub des Kolbens eine absolute Dichtheit zwischen Ringdichtung und Zylinderinnenfläche und anderseits eine möglichst kraftarme, sanfte Handhabung des Kolbens beim eigentlichen Injektionsvorgang. Diese Einwegspritzen besitzen allerdings bisher Spritzenzylinder mit kegeliger Stirnwand und zentrischem Nadelansatz, so daß wegen der speziellen Kolbenausbildung beim Spritzvorgang ein beträchtliches Restvolumen innerhalb des Spritzenzylinders verbleibt und auch das Entfernen angesaugter Luft vor einem Injizieren Schwierigkeiten bereitet, womit der Einsatzkomfort dieser Einwegspritzen wieder in Frage gestellt ist.

Gemäß der WO-A 84/02 278 gibt es zwar auch schon Spritzen mit einem Spritzenzylinder, der bei kegeliger Stirnwand einen exzentrischen Nadelansatz aufweist, und mit einem Spritzenkolben, der eine kegelige Kolbenfläche und eine stirnseitig offene Ringnut bildet, doch sind diese Spritzen kolbenformbedingt schwergärigig bzw. neigen zu Undichtheiten und die Ringnut führt wiederum zu einem unerwünschten Restvolumen und erschwert außerdem wie bei anderen Spritzen das Entlüften.

Der Erfindung liegt daher die Aufgabe zugrunde, diese Mängel zu beseitigen und eine Einwegspritze der eingangs geschilderten Art zu schaffen, die neben den Vorteilen der leichten Handhabung und der absoluten Dichtheit eine Minimierung des zurückbleibenden Restvolumens und eine Verbesserung der Entlüftungsmöglichkeit erlaubt.

Die Erfindung löst diese Aufgabe dadurch, daß die Stirnwand des Spritzenzylinders bis auf den Nadelansatzbereich eine der Kolbenkopfstirnfläche angepaßte Innenfläche bildet, wobei der Nadelansatz exzentrisch im Zwickelbereich zwischen Kragenteil und Richdichtung liegt und den Gegenzwickelbereich in der Zylinderstirnwand mit seiner Ansatzöffnung unterbricht. Durch diese gegenseitige Abstimmung von Zylinder und Kolben einerseits und die Verlagerung des Nadelansatzes in den Zwickelbereich anderseits entstehen im Stirnbereich zwischen Zylinderwand und Kolben keine Toträume mehr und der zwickelbedingte Ringraum ist direkt an die Ansatzöffnung angeschlossen, so daß das in der Spritze verbleibende Restvolumen beim Spritzvorgang praktisch auf Null reduziert und auch das Entfernen von eingesaugter Luft vor einem Injizieren wesentlich erleichtert wird.

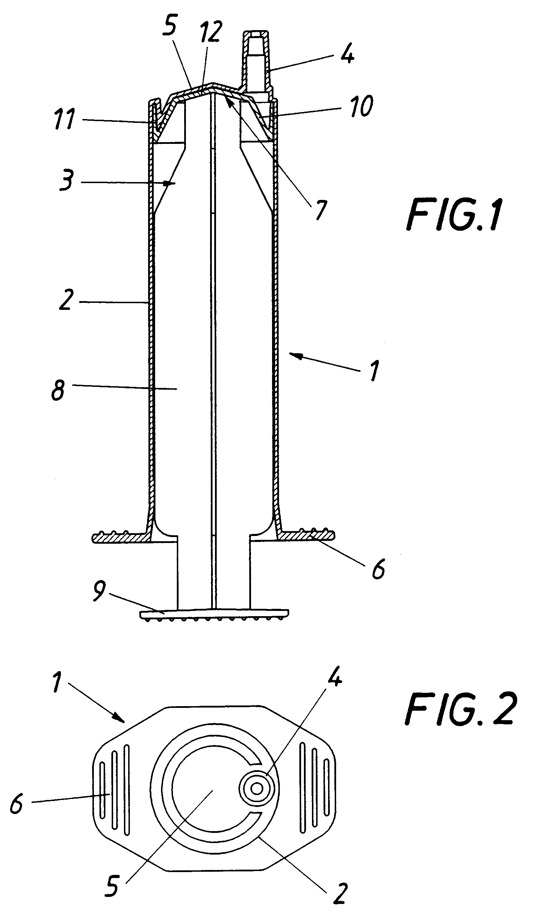

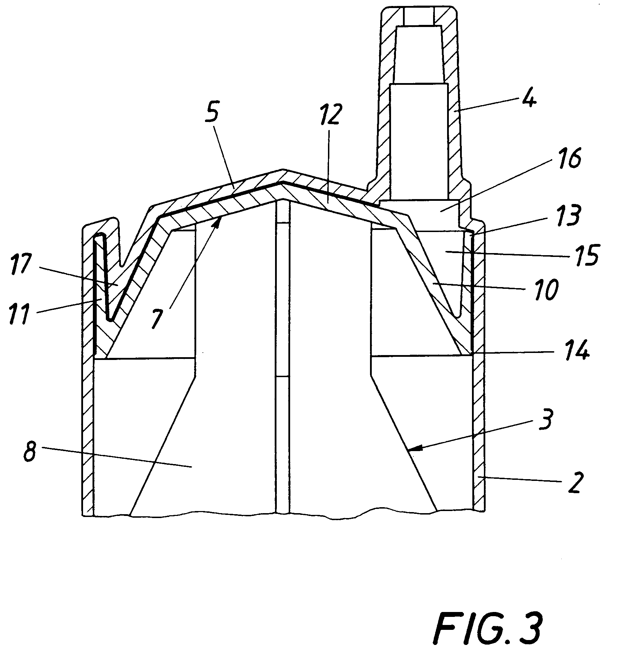

In der Zeichnung ist der Erfindungsgegenstand rein schematisch anhand eines Ausführungsbeispieles näher veranschaulicht, und zwar zeigen Fig. 1 und 2 eine erfindungsgemäße Einwegspritze im Axialschnitt und in Stirnansicht sowie Fig. 3 das stirnseitige Ende dieser Einwegspritze als Detail im Axialschnitt größeren Maßstabes.

Eine Einwegspritze 1 setzt sich aus einem Spritzenzylinder 2 und einem Spritzenkolben 3 zusammen, wobei Spritzenzylinder 2 und Spritzenkolben 3 jeweils einstückig aus thermoplastischem Kunststoff hergestellt sind. Der Spritzenzylinder 2 weist an einem Ende eine bis auf einen Nadelansatz 4 geschlossene Stirnwand 5 auf und bildet am anderen offenen Ende ein Griffstück 6. Der Spritzenkolben 3 umfaßt einen Kolbenkopf 7 und einen Kolbenschaft 8, welcher an seinem freien, aus dem Spritzenzylinder 2 herausragenden Ende eine Handhabe 9 besitzt. Der Kolbenkopf 7 trägt über einen Kragenteil 10 eine an der Zylinderinnenfläche anliegende Ringdichtung 11, wobei der Kragenteil 10 in Form eines sich zum Kolbenschaft 8 hin erveiternden Kegelstumpfes und die Ringdichtung 11 in Form eines sich vom Kragenteil kolbenkopfseitig frei vorstehenden Zylinders ausgebildet sind. Der Kolbenkopf 7 weist außerdem einen auf den Kolbenschaft 8 aufgesetzten Kegelteil 12 auf, der sich im Kragenteil 10 fortsetzt, aber einen wesentlich größeren Öffnungswinkel besitzt als dieser Kragenteil. Die Ringdichtung 11 bildet in den beiden Randbereichen ihrer Zylinderform jeweils außen umlaufende Dichtwülste 13, 14, die dichtend mit der Zylinderinnenfläche zusammenwirken. Außerdem bildet die Stirnwand 5 des Spritzenzylinders 2 bis auf den Nadelansatzbereich eine zur Kolbenkopfstirnfläche konforme Innenfläche, wobei der exzentrisch angeordnete Nadelansatz 4 im Zwickelbereich 15 zwischen Kragenteil 10 und Ringdichtung 11 liegt und hier mit seiner Ansatzöffnung 16 den Gegenzwickelbereich 17 der Zylinderstirnwand unterbricht.

Durch die besondere Ausgestaltung des Spritzenkolbens 3 ergibt sich eine federnd nachgiebige, weiche Kolbendichtung, die eine absolute Dichtheit zwischen Kolben und Zylinder gewährleist und dennoch eine leichtgängige Betätigung der Spritze erlaubt. Dabei werden durch die im Spritzenzylinder 2 auftretenden Druckverhältnisse die jeweiligen Anpreßkräfte zwischen Ringdichtung 11 und Spritzenzylinder 2 beeinflußt und an die herrschenden Druckbedingungen angepaßt. So erfolgt beim Einspritzvorgang durch den sich aufbauenden Überdruck im Zwickelbereich 15 des Kolbenkopfes 7 eine die Ringdichtung 11 radial auswärtsdrückende Kraftkomponente, wodurch der Dichtwulst 13 im freien Randbereich der Dichtung sich stärker an die Zylinderinnenfläche anlegt und die Dichtheit garantiert. Beim SaugVorgang wiederum wirkt der sich aufbauende Unterdruck im Zwickelbereich 15 auf den Kragenteil 10 ein, so daß der Dichtwulst 14 im anderen Randbereich der Dichtung 11 verstärkt gegen die Zylinderinnenwand gedrückt und auch beim Saughub die Dichtheit gewährleistet wird.

Auch eventuell auftretende dehnungsbedingte Durchmesserunterschiede des Spritzenzylinders 2 oder Verkantungen des Spritzenkolbens 3 bei der Spritzenbetätigung werden` problemlos und ohne jede Gefahr einer Undichtheit durch die Elastizität und Nachgiebigkeit der Ringdichtung 11 aufgenommen und ausgeglichen.

Die konforme Ausgestaltung von Kolbenkopfstirnfläche und Stirnwandinnenfläche verringert das beim Spritzvorgang verbleibende Restvolumen auf ein Minimum, wobei auch das Entfernen von eingesaugter Luft durch diese Konformität und die exzentrischen Nadelansatzanordnung erleichtert wird, da die gegebenenfalls vorhandenen Luftblasen dem Zwickelbereich 15 entlang in die Ansatzöffnung 16 wandern und so sicher ausgespritzt werden können.

## Patentansprüche

1. Einwegspritze (1) aus Spritzenzylinder (2) und Spritzenkolben (3), welcher einerends offene Spritzenzylinder (2) andernends eine bis auf einen Nadelansatz (4) geschlossene Stirnwand (5) aufweist und welcher Spritzenkolben (3) einen an seinem freien, aus dem Spritzenzylinder (2) herausragenden Ende eine Handhabe (9) tragenden Kolbenschaft (8) und innerhalb des Spritzenzylinders (2) einen Kolbenkopf (7) und eine über einen umlaufenden Kragenteil (10) am Kolbenkopf sitzende, an der Zylinderinnenfläche anliegende Ringdichtung (11) umfaßt, wobei der Kragenteil (10) in Form eines sich, in axialer Richtung zum Kolbenschaft (8) hin erweiternden Hohlkegelstumpfes ausgebildet ist und der radiale Abstand der Mantelinnenfläche des hohlkegelstumpfförmigen Kragenteils (10)in axialer Richtung zum Kolbenschaft hin (8) stetig zunimmt, an welchen Kragenteil (10) sich die in Form eines koaxialen Zylinders ausgebildete Ringdichtung (11), kolbenkopfseitig frei vorstehend, anschließt, dadurch gekennzeichnet, daß die Stirnwand (5) des Spritzenzylinders (2) bis auf den Nadelansatzbereich (4) eine der Kolbenkopfstirnfläche angepaßte Innenfläche bildet, wobei der Nadelansatz (4) exzentrisch im Zwickelbereich (15) zwischen Kragenteil (10) und Ringdichtung (11) liegt und den Gegenzwickelbereich (17) in der Zylinderstirnwand (5) mit seiner Ansatzöffnung (16) unterbricht.

## Claims

1. A single-use syringe (1) consisting of a syringe cylinder (2) and a syringe piston (3), which has,at one end, an open syringe cylinder (2) and, at the other end, a front wall (5) which is closed off as far as a needle attachment piece (4), said piston comprising, at its free end which protrudes from the syringe cylinder (2), a piston shaft (8) bearing a handle (9) and, within the syringe cylinder (2), a piston head (7) and an annular seal (11) located in contact with the inner surface of the cylinder and mounted on the piston head by means of an encompassing collar component (10), wherein the collar component (10) has the shape of an obtuse-angled hollow cone extending in an axial direction towards the piston shaft (8) and the radial clearance of the inner surface of the wall of the obtuse-angled hollow cone-shaped collar component (10) continuously increases in an axial direction towards the piston shaft (8), to which said collar component (10) there is connected the annular seal (11) which has an open face projection on the cylinder head side and which is in the form of a coaxial cylinder, characterised in that the front wall (5) of the syringe cylinder (2) as far as the needle attachment piece region (4) comprises an inner surface matching the front surface of the piston head, wherein the needle attachment piece (4) lies eccentrically in the inset wedge zone (15) between the collar component (10) and the annular seal (11) and interrupts with its attachment aperture (16) the counter-inset wedge zone (17) in the front wall (5) of the cylinder.

## Revendications

1. Seringue jetable (1) constituée d'un cylindre de seringue (2) et d'un piston de seringue (3), qui présente à une extrémité un cylindre de seringue (2) ouvert et à une autre extrémité une paroi frontale (5) fermée allant jusqu'à un appendice à aiguille (4) et le piston de seringue (3) comprenant une tige de piston (8) portant à son extrémité libre, sortant du cylindre de seringue (2) une gâchette (9) et, à l'intérieur du cylindre de seringue (2) une tête de piston (7) et un joint d'étanchéité annulaire (11), appuyant sur la surface intérieure du cylindre et reposant, par l'intermédiaire d'une partie collerette (10) faisant le pourtour, sur la tête de piston, la partie collerette (10) étant réalisée sous la forme d'un tronc de cône creux allant en s'agrandissant en direction axiale en direction de la tige de piston (8) et l'espacement radial entre la surface intérieure d'enveloppe de la partie collerette (10) de forme tronconique creuse allant en augmentant constamment en direction axiale, en direction de la tige de piston (8), le joint d'étanchéité annulaire (11) réalisé sous la forme d'un cylindre coaxial se raccordant, en faisant saillie librement du côté de la tête de piston, à la partie collerette (10), caractérisée en ce que la paroi frontale (5) du cylindre de seringue (2) constitue jusqu'à la zone d'appendice à aiguille (4) une surface intérieure adaptée à la face frontale de la tête de piston, l'appendice à aiguille (4) étant situé de façon excentrée dans la zone de gousset (15) entre la partie collerette (10) et le joint d'étanchéité annulaire (11) et interrompant par son ouverture d'appendice (16) la zone de gousset conjuguée (17) ménagée dans la paroi frontale de cylindre (5).
